# FASCICULE DE BREVET EUROPEEN

(11) **EP 1 048 672 B1**
(45) Date de publication et mention de la délivrance du brevet: **04.09.2002**
(21) Numéro de dépôt: 00401125.0
(22) Date de dépôt: 21.04.2000
(51) Int. Cl.: C07H 3/02

(54) **Procédé de préparation de fructose par hydrogénation de la glucosone**
Verfahren zur Herstellung von Fructose durch die Hydrierung von Glucosone
Process for the preparation of fructose by the hydrogenation of glucosone

(30) Priorité: 27.04.1999 FR 9905309
(43) Date de publication de la demande: 02.11.2000
(73) Titulaire: Roquette Frères, 62136 Lestrem (FR)
(72) Inventeur: Choque, Jean-Christophe, 59000 Lille (FR); Fleche, Guy, 59190 Hazebrouck (FR)
(74) Mandataire: Boulinguiez, Didier

(56) Documents cités:
- WO-A-81/03666
- US-A- 4 246 347

## Description

L'invention concerne un procédé de préparation de fructose par hydrogénation catalytique d'une solution de glucosone à teneur élevée en matière sèche en mettant en oeuvre des conditions de pression et de température particulières. L'hydrogénation de la glucosone peut ainsi s'effectuer de manière continue en lit fixe de catalyseur, et plus particulièrement en une succession de lits fixes de catalyseur disposés en série et en deux zones de réaction.

La préparation industrielle du fructose met en oeuvre classiquement un procédé en deux étapes, consistant à produire du glucose par hydrolyse de polysaccharides tels que l'amidon ou des dérivés de l'amidon, puis à conduire l'isomérisation du glucose ainsi obtenu en fructose.

Les compositions dites à haute teneur en fructose ainsi obtenues renferment cependant pratiquement encore autant de glucose contaminant que de fructose. Ainsi, il est décrit des compositions dites à haute teneur en fructose, obtenues par isomérisation du glucose, constituées de 42 % en poids de fructose, avec 50 % en poids de glucose, et environ 8% en poids de polysaccharides résiduels.

Il est en outre communément admis que l'isolement du fructose à partir desdites compositions est difficile à réaliser, et nécessite l'utilisation de techniques de purification lourdes et coûteuses.

Une technique alternative à la préparation du fructose par isomérisation du glucose, est une technique qui consiste à mettre en oeuvre deux étapes, la première consistant à convertir le glucose en glucosone (également connue sous les noms de 2 céto-glucose ou de D-arabino-2-hexulosone), la seconde consistant à conduire la réduction chimique ou enzymatique de la glucosone ainsi obtenue en fructose.

Dans cette première étape, la conversion du glucose en glucosone peut être effectuée par voie chimique, (par traitement à l'eau oxygénée ou avec de l'acétate de cuivre) ou avantageusement par voie enzymatique (à l'aide d'une pyranose 2-oxydase) afin de conduire à un produit de haute pureté.

Dans la seconde étape, il a été proposé de convertir la glucosone en fructose par une technique de réduction par voie chimique (par exemple en présence de zinc et d'acide acétique) ou par voie enzymatique (à l'aide d'une réductase, comme par exemple décrit dans KIESLICH, ed., Microbial Transformation of non-steroid cyclic compounds, Georg Threme Publishers, Stuttgart (1976), pp 279-280).

Cependant, les procédés de réduction chimique ou enzymatique de la glucosone conduisent à la fabrication de coproduits qui contaminent le fructose ainsi obtenu.

Ainsi, il est par exemple connu que la voie chimique de réduction de la glucosone génère des halohydrines et des époxydes hautement toxiques. Il est donc nécessaire d'avoir recours à des techniques de purification coûteuses pour préparer un fructose de pureté satisfaisante, par exemple de qualité alimentaire.

Il a ensuite été proposé de mettre en oeuvre une technique d'hydrogénation catalytique de la glucosone.

Les brevets US 4.246.347 et US 4.423.149 décrivent un procédé de préparation de fructose à partir du glucose, où la glucosone, obtenue par conversion enzymatique du glucose à l'aide d'une pyranose 2-oxydase, est soumise à une hydrogénation en présence d'un catalyseur approprié.

Plus particulièrement, la technique décrite consiste à réaliser une hydrogénation de la solution de glucosone à très faible matière sèche, en mode discontinu, en présence d'un catalyseur préférentiellement constitué de 5 % de palladium sur charbon, à pression atmosphérique et température ambiante.

Si cette technique permet d'assurer une conversion essentiellement complète de la glucosone en fructose, la richesse finale n'est au maximum que de 95 %, avec une productivité médiocre. De plus, seules des solutions de glucosone de matière sèche comprise entre 0,2 et 1,4 % en poids sont réellement mises en oeuvre.

Une alternative à ce procédé d'hydrogénation catalytique est décrite dans la demande de brevet WO 81.03666, où la solution de glucosone, toujours à faible matière sèche, est hydrogénée dans des conditions de pression et de température élevées, en présence de catalyseur au Ni de Raney. Il est ainsi choisi de conduire l'hydrogénation catalytique en mode discontinu à une température comprise entre 100 et 150°C, et à une pression de 500 bars.

Si la productivité en fructose est améliorée, ce procédé présente cependant l'inconvénient de nécessiter la mise en oeuvre de pressions très importantes, et il n'est décrit que pour la conversion de la solution de glucosone à faible matière sèche (de l'ordre de 2,5 % en poids).

Il apparaît donc clairement dans l'état de la technique que les procédés d'hydrogénation en mode discontinu de la glucosone ne permettent pas d'obtenir du fructose avec une sélectivité et une productivité élevées pour un taux de conversion élevé. De plus, aucun de ces procédés d'hydrogénation n'est décrit pour convertir la solution de glucosone à haute matière sèche, et n'est donc industriellement satisfaisant.

L'invention a donc pour but de remédier à cette situation, et de proposer un moyen permettant d'atteindre un excellent compromis entre pureté, taux de conversion, sélectivité et productivité, et ce pour une matière sèche élevée en glucosone.

La société Demanderesse a trouvé, après de nombreuses recherches, qu'un tel moyen pouvait consister en un procédé d'hydrogénation catalytique de la glucosone à haute matière sèche, conduite dans des conditions particulières de température et de pression.

La présenté invention a précisément pour objet un procédé de préparation de fructose par hydrogénation de la glucosone, caractérisé par le fait que l'on conduit l'hydrogénation en présence d'un catalyseur et en soumettant une solution de glucosone présentant une matière sèche au moins égale à 10 % en poids, de préférence comprise entre 20 et 50 % en poids, à une pression au moins égale à 20 bars, de préférence comprise entre 30 et 150 bars, et à une température au moins égale à 50°C, de préférence comprise entre 50 et 150°C.

Le procédé conforme à l'invention met en oeuvre une solution de glucosone présentant une matière sèche au moins égale à 10 % en poids, de préférence comprise entre 20 et 50 % en poids.

On choisit de préparer la solution à hydrogéner avec de la glucosone de très haute pureté, avantageusement produite par voie enzymatique à partir du glucose par tout moyen connu de l'homme du métier.

On entend par « haute pureté », une teneur en glucosone de l'ordre de 100 %.

Le catalyseur est choisi dans le groupe constitué du palladium, du nickel, du ruthénium, du platine, du rhodium, du cobalt, du cuivre, du zinc, du chrome, du manganèse, du tungstène, et est de préférence du palladium ou du nickel (sous forme Ni de Raney), et encore plus préférentiellement du palladium.

Le procédé conforme à l'invention permet alors, de manière surprenante et inattendue, de mettre en oeuvre les catalyseurs palladium et nickel de Raney dans les mêmes conditions de pression et de température d'hydrogénation.

Il est en effet décrit dans l'état de la technique des conditions de pression et de température relativement basses (atmosphérique et ambiante) pour conduire l'hydrogénation de la glucosone avec un catalyseur au palladium, alors que la catalyse au nickel de Raney demande des pressions de l'ordre de 500 bars et plus, et des températures élevées.

Dans le procédé conforme à l'invention, le catalyseur est généralement imprégné ou co-échangé sur un support inerte, de préférence choisi dans le groupe constitué du charbon actif, de la tourbe, des zéolites, des aluminosilicates, du dioxyde de titane. De préférence il est constitué de charbon actif.

Le rapport pondéral catalyseur / support inerte est établi avantageusement à une valeur comprise entre 1 et 5 %, de préférence de l'ordre de 5 %, comme exemplifié ci-après.

Il est également possible de mettre en oeuvre un catalyseur comportant un agent promoteur. Cet agent promoteur peut être choisi dans le groupe constitué du titane, du molybdène et du platine.

Dans le procédé conforme à l'invention, on choisit de mettre en oeuvre une pression d'hydrogénation au moins égale à 20 bars, de préférence comprise entre 30 et 150 bars, et une température au moins égale à 50°C, de préférence comprise entre 50 et 150°C.

Dans un mode de réalisation du procédé conforme à l'invention, on choisit de conduire l'hydrogénation en mode continu sur lit fixe de catalyseur.

Le catalyseur est alors disposé dans un lit fixe sous la forme d'un empilement compact de particules, le tout placé sur des grilles de soutien dans un réacteur d'hydrogénation. On choisit avantageusement un réacteur à ruissellement.

Au sens de l'invention, on entend par « réacteur à ruissellement », un réacteur d'hydrogénation dans lequel une phase liquide contenant le produit à hydrogéner et une phase gaz circulent de manière co-courante ou contre-courante, de préférence de manière co-courante de haut en bas, dans un lit fixe de particules de catalyseur où s'effectue la réaction d'hydrogénation.

Les débits d'écoulement de ces deux phases sont réglés pour permettre au liquide de ruisseler sur lesdites particules de catalyseur, et assurer le meilleur contact entre les deux phases liquide et gazeuse d'une part, et la phase solide du catalyseur d'autre part.

Dans un mode de réalisation du procédé conforme à l'invention, on choisit de préparer un lit fixe constitué de 200 1 de catalyseur commercial, un débit d'alimentation de la solution de glucosone, d'une matière sèche comprise entre 10 et 50 % en poids, à une valeur comprise entre 150 et 250 kg/h, et une quantité d'hydrogène introduite dans ledit réacteur à ruissellement de l'ordre de cinq à quinze fois la stoechiométrie de la réaction.

Les pression et température d'hydrogénation mises en oeuvre sont alors avantageusement choisies à des valeurs respectives de l'ordre de 30 à 120 bars, par exemple 50 bars, et de l'ordre de 100 à 150°C, par exemple 130°C.

Dans un mode préférentiel de réalisation du procédé conforme à l'invention, on choisit de mettre en oeuvre un procédé continu d'hydrogénation de la glucosone dans une succession de lits fixes de catalyseur disposés en série et en au moins deux zones de réaction.

L'hydrogénation est alors avantageusement conduite dans une première zone de réaction, constituée d'au moins un lit fixe de catalyseur, de manière à obtenir une sélectivité élevée en fructose, puis dans une seconde zone de réaction, constituée d'au moins un lit fixe de catalyseur, où l'hydrogénation est conduite de manière à obtenir, à sélectivité élevée, une conversion totale en fructose.

On peut mettre en oeuvre des catalyseurs de nature différente dans chacune des zones de réaction. Cependant on préfère utiliser un catalyseur de même nature dans ces deux zones.

Dans la première zone de réaction, les conditions de pression et de température sont réglées de manière à obtenir une sélectivité élevée, de l'ordre de 100 %. On choisit une pression au moins égale à 50 bars, et une température au moins égale à 100°C, de préférence comprise entre 100 et 150°C, comme exemplifié ci-après.

Dans la seconde zone de réaction, les conditions de pression et de température sont réglées pour obtenir un taux de conversion élevé, de l'ordre de 100 %. On choisit une pression au plus égale à 150 bars, de préférence comprise entre 50 et 100 bars, et une température au plus égale à 100°C, de préférence comprise entre 50 et 100°C, comme exemplifié ci-après.

Les lits fixes de catalyseur sont avantageusement disposés dans des réacteurs à ruissellement. On choisit de mettre en oeuvre 200 1 de particules de catalyseur commercial, un débit d'alimentation de la solution de glucosone d'une matière sèche comprise entre 10 et 50 % en poids à une valeur comprise entre 200 et 400 kg/h, et une quantité d'hydrogène comprise entre cinq et quinze fois la stoechiométrie de la réaction.

D'autres caractéristiques et avantages de l'invention apparaîtront à la lecture de l'exemple non limitatif décrit ci-dessous.

### Exemple

La réaction d'hydrogénation est effectuée dans deux réacteurs à ruissellement connectés en série avec un échangeur thermique intermédiaire.

Les deux réacteurs renferment chacun un unique lit fixe de catalyseur au palladium sur charbon, dans lequel on fait circuler l'hydrogène et la solution de glucosone de manière co-courante du haut vers le bas desdits réacteurs.

Chaque lit fixe de catalyseur au palladium est constitué d'un empilement compact de grains de catalyseur cylindriques.

Chaque grain de catalyseur est composé de charbon actif de type NORIT RX08, sous la forme d'un cylindre de 0,8 mm de diamètre et de 1 à 5 mm de longueur, contenant 5 % en poids de palladium.

Chaque réacteur contient de l'ordre de 200 1 de catalyseur, disposé en lit fixe de 30 cm de diamètre et de l'ordre de 3 m de hauteur.

On alimente simultanément la première zone d'hydrogénation avec une solution de glucosone à 30 % de matière sèche, à un débit de 300 kg/h et de l'hydrogène à raison de 15 kg/h.

La pression de fonctionnement dans le premier réacteur est de 50 bars, et la température est fixée à 130°C.

La température d'entrée du second réacteur est ramenée à 100°C, et la pression est de 100 bars.

En sortie du deuxième réacteur, la sélectivité est de 96 %, avec un taux de conversion de 100 %.

## Revendications

1. Procédé de préparation de fructose par hydrogénation de la glucosone, **caractérisé par le fait que** l'on conduit l'hydrogénation en présence d'un catalyseur et en soumettant une solution de glucosone présentant une matière sèche au moins égale à 10 % en poids, de préférence comprise entre 20 et 50 % en poids, à une pression au moins égale à 20 bars, de préférence comprise entre 30 et 150 bars, et à une température au moins égale à 50°C, de préférence comprise entre 50 et 150°C.

2. Procédé selon la revendication 1, **caractérisé par le fait que** le catalyseur est choisi dans le groupe constitué du palladium, du nickel, du ruthénium, du platine, du rhodium, du cobalt, du cuivre, du zinc, du chrome, du manganèse, du tungstène, et est de préférence du palladium ou du nickel.

3. Procédé selon l'une ou l'autre des revendications 1 et 2, **caractérisé par le fait que** l'on conduit l'hydrogénation de la glucosone en fructose en mode continu sur lit(s) fixe(s) de catalyseur.

4. Procédé selon la revendication 3, **caractérisé par le fait que** l'hydrogénation continue est conduite sur au moins un lit fixe de catalyseur dans lequel la pression est comprise entre 30 et 120 bars et la température est comprise entre 100 et 150°C.

5. Procédé selon l'une ou l'autre des revendications 3 et 4, **caractérisé par le fait que** l'on conduit l'hydrogénation continue de la glucosone en fructose dans une succession de lits fixes de catalyseur disposés en série qui comprend :
- une première zone d'hydrogénation, constituée d'au moins un lit fixe de catalyseur, où la pression est au moins égale à 50 bars et la température est au moins égale à 100°C, de préférence comprise entre 100 et 150°C,
- une seconde zone d'hydrogénation, constituée d'au moins un lit fixe de catalyseur, où la pression est au plus égale à 150 bars, de préférence comprise entre 50 et 100 bars, et la température au plus égale à 100°C, de préférence comprise entre 50 et 100°C.

## Patentansprüche

1. Verfahren zur Herstellung von Fructose durch Hydrierung von Glucoson, **dadurch gekennzeichnet, dass** man die Hydrierung in Gegenwart eines Katalysators durchführt, und indem man eine Glucosonlösung mit einer Trockenmasse von mindestens gleich 10 Gew.-%, vorzugsweise zwischen 20 und 50 Gew.-%, einem Druck von mindestens gleich 20 bar, vorzugsweise zwischen 30 und 150 bar, und einer Temperatur von mindestens gleich 50°C, vorzugsweise zwischen 50 und 150°C, aussetzt.

2. Verfahren nach Anspruch 1, **dadurch gekennzeichnet, dass** der Katalysator aus der Gruppe ausgewählt wird, die aus Palladium, Nickel, Ruthenium, Platin, Rhodium, Kobalt, Kupfer, Zink, Chrom, Mangan, Wolfram besteht, und vorzugsweise Palladium oder Nickel ist.

3. Verfahren nach einem der Ansprüche 1 und 2, **dadurch gekennzeichnet, dass** man die Hydrierung von Glucoson zu Fructose kontinuierlich auf Katalysatorfestbett/en durchführt.

4. Verfahren nach Anspruch 3, **dadurch gekennzeichnet, dass** die kontinuierliche Hydrierung auf mindestens einem Katalysatorfestbett durchgeführt wird, in dem der Druck zwischen 30 und 120 bar und die Temperatur zwischen 100 und 150°C beträgt.

5. Verfahren nach einem der Ansprüche 3 und 4, **dadurch gekennzeichnet, dass** man die kontinuierliche Hydrierung von Glucoson zu Fructose in einer Folge von in Reihe angeordneten Katalysatorfestbetten durchführt, die umfasst:
- eine erste Hydrierungszone, die aus mindestens einem Katalysatorfestbett besteht, in dem der Druck mindestens gleich 50 bar ist und die Temperatur mindestens gleich 100°C ist und vorzugsweise zwischen 100 und 150°C beträgt,
- eine zweite Hydrierungszone, die aus mindestens einem Katalysatorfestbett besteht, in dem der Druck höchstens gleich 150 bar ist und vorzugsweise zwischen 50 und 100 bar liegt und die Temperatur höchstens gleich 100°C ist und vorzugsweise zwischen 50 und 100°C liegt.

## Claims

1. Process for preparing fructose by the hydrogenation of glucosone, **characterized by** the fact that the hydrogenation is conducted in the presence of a catalyst and by submitting a solution of glucosone having a dry matter of at least 10% by weight, preferably of between 20 and 50% by weight, to a pressure of at least 20 bars, preferably of between 30 and 150 bars, and to a temperature of at least 50°C, preferably of between 50 and 150°C.

2. Process according to claim 1, **characterized by** the fact that the catalyst is chosen among the group consisting of palladium, nickel, ruthenium, platinum, rhodium, cobalt, copper, zinc, chromium, manganese, tungsten, and is preferably palladium or nickel.

3. Process according to one or the other of claims 1 and 2, **characterized by** the fact that the hydrogenation of glucosone into fructose is conducted in fixed catalyst bed(s) on a continuous mode.

4. Process according to claim 3, **characterized by** the fact that the continuous hydrogenation is conducted in at least one fixed catalyst bed wherein the pressure is of between 30 and 120 bars and the temperature of between 100 and 150°C.

5. Process according to one or the other of claims 3 and 4, **characterized by** the fact that the continuous hydrogenation of glucosone into fructose is conducted in a succession of fixed catalyst beds disposed in series which comprises:
- a first hydrogenation zone consisting of at least one fixed catalyst bed wherein the pressure is of at least 50 bars and the temperature of at least 100°C, preferably of between 100 and 150°C,
- a second hydrogenation zone consisting of at least one fixed catalyst bed wherein the pressure is of at most 150 bars, preferably of between 50 and 100 bars, and the temperature of at most 100°C, preferably of between 50 and 100°C.
